# EUROPEAN PATENT SPECIFICATION

(11) **EP 1 820 465 B1**
(45) Date of publication and mention of the grant of the patent: **07.04.2010**
(21) Application number: 06003497.2
(22) Date of filing: 21.02.2006
(51) Int. Cl.: A61B 19/00

(54) **Universal image registration interface**
Universelle Schnittstelle zur Registrierung von Bildern
Interface universelle d'enregistrement d'images

(43) Date of publication of application: 22.08.2007
(73) Proprietor: BrainLAB AG, 85622 Feldkirchen (DE)
(72) Inventor: Vilsmeier, Stefan, 80539 München (DE); Bauch, Thomas, 85232 Bergkirchen (DE); Mezger, Uli, 81543 München (DE)
(74) Representative: Schwabe - Sandmair - Marx

(56) References cited:
- WO-A2-2004/100767
- US-A- 5 971 997
- US-A1- 2001 007 918
- US-A1- 2003 187 351
- US-A1- 2004 167 654

## Description

The present invention relates to a method for supporting the treatment of a patient by means of a navigation system. Such navigation systems detect and track the positions of patients, parts of the patient's body, and targets of treatment as well as of treatment devices, and show, in many cases, the surgeon concerned images on a monitor, which he uses to support his treatment.

For a navigation system to be able to show the correct images to the surgeon, it is necessary to correlate the images with the actual patient space, this process is called registration.
The idea of feature or object based matching based on, say, fiducials or anatomical landmarks, to register a patient can be found in nearly all image guided surgery products. These methods basically require 'easy-to-identify' structures in the image set of the patient (usually MR or CT based tomography images) and 'easy-to-reach' structures rigidly attached to the patient, allowing to correlate the image set space with the actual patient orientation in the operating room.

'Easy-to-identify' structures in image sets are usually, but not restricted to, spherical markers or rods with a high contrast, which are attached to the patient in such a way that they are also 'easy-to-reach' with a tracked instrument of the image guided surgery (IGS) system. The quality of the registration thereby always depends on the expertise of the person applying the right amount of markers or rods in the right configuration and the individual setup of the patient bedding, which limits access to markers or rods. It is not always guaranteed that structures are 'easy-to-identify', because they can be ambiguous and corresponding structures are not always 'easy-to-reach', because of the patient bedding and specific operating room setup, as e.g. drapes, tubes etc.

Hence a consistent registration quality using conventional feature or object based matching, especially when based on fiducials as registration markers, is not guaranteed.

In comparison to automatic registration methods, the time to attach and identify these registration markers is usually high and therefore not feasible for intraoperative use. On the other hand, the disadvantage of automatic registration methods is that they are proprietary and require a strong integration in the navigation software.

In contrast, standard paired-point matching image registration, one embodiment of feature or object based matching, is available with typically any commercially available IGS system. In this case, however, accuracy might be low and one needs to access the patient anatomy for image registration. This is typically not possible for the registration of intraoperatively acquired images, because the patient anatomy is not accessible because of the draping.

But the registration of intraoperative images is thereby especially critical for navigations system because problems may arise in the course of treatment if the tissue is subjected to shifting during treatment, as may happen, for example, due to liquid discharge or removal of tissue. In such a situation, i.e. if the target of treatment or the surrounding tissue together with the target of treatment has been shifted, the supporting navigation becomes inaccurate, with the result that the surgeon involved is again entirely left to his own observations or, if he did not notice the shift, might possibly operate at wrong positions.

A method for supporting the treatment of a patient is described in US 2001/0007918 A1, disclosing a method for registering a body structure, wherein
- a reference structure comprising an imaging structure (8) having a plurality of connection systems (4) for MR markers and a plurality of IR markers (2), wherein the IR markers (2) are separately arranged from the connection systems (4) and have a known relationship to the imaging structure (8), has been attached to the body;
- an imaging of the body or body structure is performed to obtain an image and the relationship between the body structure and MR markers attached to the connection systems (4);
- the position of the IR markers (2) is identified; and
- the position of the body structure in space is identified using the identified position of the IR markers (2) and the known relationship between the IR markers (2) and the MR markers (3).

US 2004/0167654 A1 refers to a system for registering an orthopedic implant in a computer assisted navigation system. The system includes a plurality of differently sized implants which may be the femoral component or hip stem of a prosthetic hip joint. A registration device is engageable with each of the implants in a predefined relative position. The registration device also includes at least one reference element registerable in the computer assisted navigation system. A second reference structure also having at least one reference element registerable in the computer assisted navigation system is detachably secured to the implant. The relative positions of the reference elements located on the registration device and second reference structure differs for each of the plurality of implants and thereby allows the navigation system to determine the nominal size of the implant. The relative position and orientation of the implant relative to the second structure can also be calibrated using the registration device.

US 5,971,997 refers to an apparatus for calibrating and recalibrating a digitized navigator or so-called frameless stereotactic instrument or device during a surgical operation. The apparatus enables calibration for the stereotactic digitizer to be recovered after sterile draping of the patient, movement of the patient's head during operation, relative movement of the patient's head and reference apparatus associated with the stereotactic digitizer, power failures, or other events which could compromise calibration of the stereotactic digitizer during the operation which would otherwise preclude continuations using the digitizer. It also provides convenient means for initial calibration or repeat calibration, should that be necessary. The apparatus is, in one embodiment, cooperatively coupled to, or coupled so as to move with, the portion of the patient's body which is being operated upon and enables the repositioning of the recalibration means after sterile draping. In exemplary embodiments, the apparatus would include a reference point structure or a reference holding structure that can be attached to, for example, a surgical head clamp in a neurosurgical operation and can be accessed for calibration positioning or calibration locationing by the digitizer during the operation. The apparatus could be removed, sterilized, and repositioned in an identical position relative to the head clamp, and therefore the patient's head during the operation, assuring that sterility and loss of calibration would not be compromised.

WO 2004/100767 A2 refers to a fiducial marker holder apparatus including a maxillary holding device and an open-ended frame. The maxillary holding device is configured to be temporarily secured to only a maxillary-region of a patient. The open-ended frame has first and second arms and is configured to be removably attached to the maxillary holding device. The first arm has at least one marker attachment point that receives fiducial markers and the second arm has a plurality of marker attachment points that receive fiducial markers. At least two of the marker attachment points of the second arm are configured to receive fiducial markers in different orientations with respect to the open-ended frame and each other.

US 2003/0187351 A1 refers to a method and apparatus for positioning a surgical instrument during stereotactic surgery on a body, for example, during spinal surgery or general surgery. A mounting device is attached to a bone structure of the body, for example, by attaching a rail to the spine for spinal surgery, or a mounting plate and a rod attached to the mounting plate. In spinal surgery, the rail is attached to the spine by clamping the rail to two spinous processes and adjusting the separation of the two spinous processes to match the separation of the two processes in three-dimensional scanned image, thereby matching a curvature of the spine with a corresponding curvature of the spine in the image. Multiple of fiducial points are located on the body in relation to the mounting device. An adjustable guidance fixture that includes an instrument guide for guiding the surgical instrument along a constrained trajectory relative to the instrument guide is attached to the mounting device. A location and orientation of the instrument guide is tracked and a position of the constrained trajectory in the three-dimensional image is computed. The guidance fixture is adjusted relative to the mounting device using a first constrained motion followed by a second constrained motion, so that the constrained trajectory of the instrument guide passes through the target.

It is therefore an object of the present invention to provide the treating surgeon with a method for registering a body structure enabling him to still navigate precisely by means of a navigation system even after tissue has been shifted during an operation without having to make software modifications to the proprietary IGS system.

According to the invention, this object is solved by a method as set forth in claim 1. An advantageous embodiment of the invention is described in the dependent claim.

A generic and universal interface for any third party IGS system is described to easily register pre- and intraoperative patient data without the need of accessing the anatomical structures of the patient and the need of any software modifications to the proprietary IGS system.

The automatic image registration method according to the invention is a straight-forward method to register pre- and intraoperative images with high accuracy without accessing anatomical structures of the patient and without manual identification of scan or registration markers. This advantage is achieved by providing proprietary hardware and software and implementing specific software algorithms for marker detection and coordinate transformation into the navigation software. The image registration information is thus available for the proprietary image format.

A generic interface for any third party IGS system allows to easily register pre- and intraoperative patient data without the need of accessing the anatomical structures of the patient and the need of any software modifications to the third party IGS system. The output of the generic interface is an image set in standard image format (e.g. DICOM) with fixed drawn-in registration markers that can be easily identified by any third party IGS system using for example paired-point-matching registration.

The advantages using such a registration structure and methodology are:
- Registration quality does not depend on user expertise
- Reliable due to redundant information
- Independent of patient bedding
- Consistent registration quality
- Guaranteed 'Easy-to-identify' structures in images
- Guaranteed 'Easy-to-reach' structures for registration
- Time efficient, since only the minimal amount of points need to be identified using a tracked instrument
- Requires no explicit software integration into existing IGS systems
- Customer can use one hardware for different proprietary IGS systems
- Allows intraoperative registration

The further advantages of the method in accordance with the invention are based on the fact that, in addition to the data record the navigation system has used up to that moment, one or several further current patient data records are created, either automatically or on demand, by an image-generating method, and that each current data record is integrated into the navigation system in a computer-aided manner. It is, thus, not only ensured that a new and current data record, recording the cited tissue shifts and changes, is available at a given time, but the current data record will simultaneously be linked or integrated into the navigation system so that the surgeon concerned is able to continue his work quickly with the assistance of a precisely integrated and updated navigation support. Accordingly, incorrect treatment can be avoided, and the surgeon concerned no longer has to depend on visually perceiving large-scale tissue removals or liquid discharges.

The other data record(s) of the patient can be created during the operation by means of different methods. These are, in particular, magnetic resonance tomography (MR), computer tomography (CT) or the SPECT / PET methods.

In a first specific embodiment of the method according to the invention, a reference structure is positioned at the patient or in the surrounding area of the target of treatment while the current data record is being created, said reference structure comprising artificial markers, e.g. notches or other structures, which can be detected by the proprietary navigation system e.g. by pointing at them with a tracked instrument as well as markers which can be detected by the image-generating method, the assignment of data for the markers resulting in a positional integration of the current data record into the navigation system.

An artificial marker is any structure or identifiable element which can be used to identify the position of this artificial marker in space, e.g. by using a trackable pointer pointing sequentially at these artificial markers.

According to the present invention, the artificial markers are notches.

Accordingly, the reference structure and the notches or artificial markers thereof are the point of intersection for the assignment of the current data record into the navigation system. Due to the positional detection of the artificial navigation system marker, e.g. by pointing at several notches, the navigation system knows the position of the reference structure and, furthermore, its position is known in the newly created data record, as here markers are also detected at the reference structure. Similarly, the image-generating system also knows the position of the pixels and can detect/compensate the deviations between the individual data records of the patient (caused by different positions of the patient) and/or transmit them to the navigation system. Thus, each subsequently following data record is automatically referenced, i.e. only the first data record has to be localized/referenced, provided that the patient is firmly fixed, e.g. by means of a rigid head fixation.

The invention is described further by reference to embodiments shown in the drawings, wherein the Figures show:
- Fig. 1: a reference structure ;
- Fig. 2: the abstract model of the reference structure shown in Fig. 1;
- Fig. 3: the workflow of a software module for referencing the imaged structure; and
- Fig. 4: an embodiment of the inventive method.

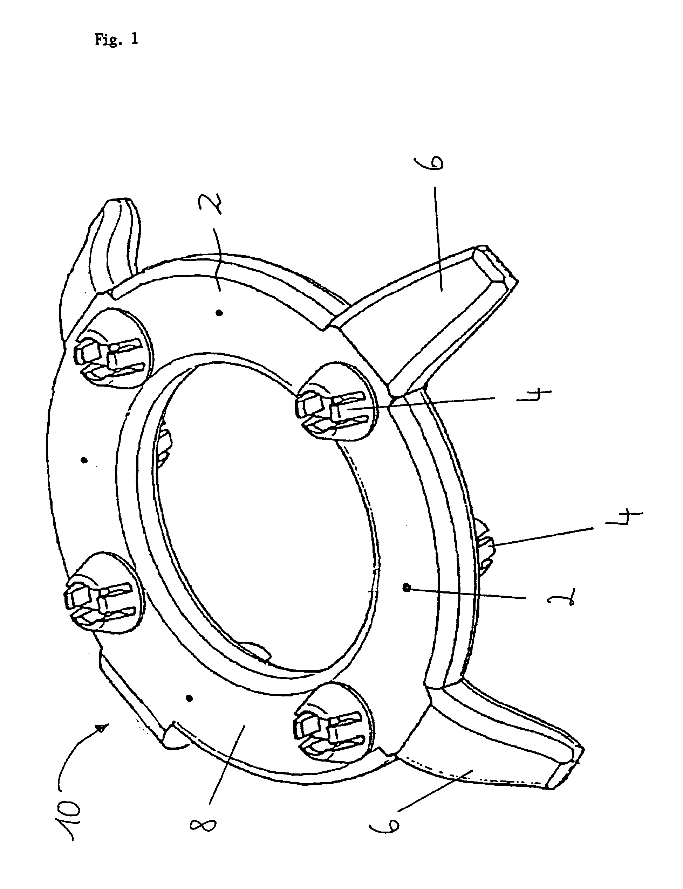

Figure 1 shows a reference structure 10 as it can be used to integrate the position of an updated data record into a navigation system. In this embodiment the reference structure 10 comprises a carrier ring 8 with arms 6. Said carrier ring 8 includes artificial markers, being notches 2, as well as fixtures 4 for MR or CT markers (not shown), which are visible in a magnetic resonance or computer tomographic image. These fixtures 4 are provided on both the top and the bottom side of said ring 8 to receive ball-shaped MR / CT markers therein. It can be assumed that all conceivable tracking systems can be used within the scope of the present invention, i.e. for example also those comprising actively radiating markers, magnetic systems or systems based on ultrasonics. MR or CT visible markers or structures of different shape may also be used, e.g. rods, flat or cornered markers.

The use of such a reference structure will now be explained in detail with regard to the course of a treatment using navigation updating according to the invention.

First of all, a diagnostic image data record of a patient is created, e.g. a magnetic resonance or computer tomography data record. Then, the patient is moved into the operating theater where, in advance, the first data record may again be adjusted by means of known adjusting methods so as to make a correctly referenced initial data record available for navigation.

Now, the surgeon concerned performs the image-guided operation with the assistance of the navigation system. Should the surgeon find out in the course of the operation that a great amount of tissue liquid has already been drained off or that tissue has already been removed to such an amount that inaccuracies occur in the navigation system due to the shift of tissue, he can activate the intra-operative navigation updating in accordance with the present invention. To do so, the patient is, first of all, covered with sterile cloth. If the operation is to be performed in the region of the head, a sterilized reference structure 10 can be placed, for example, on the patient's face, and the generation of the current data record be started. To do so, a mobile MR or CT device, for example, is moved into the operating theatre and positioned beside the patient's head. Or an already integrated MR or CT within the operating theatre is used.

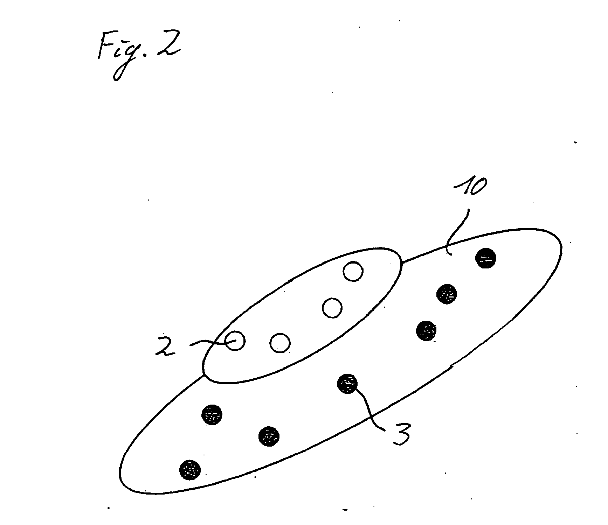

In the case of the intra-operative MR/CT scan, the MR/CT markers 3 of fixtures 4 are also scanned. Where the hardware module, as an abstract embodiment of the registration structure of Figure 1, is shown in Fig. 2 and comprises an array of unambiguously arranged, 'easy-to-detect' spherical markers 3 and comprises an array of 'easy-to-reach' notches 2. The relation between the registration structure markers 3 and the notches 2 is known either by construction or measurement.

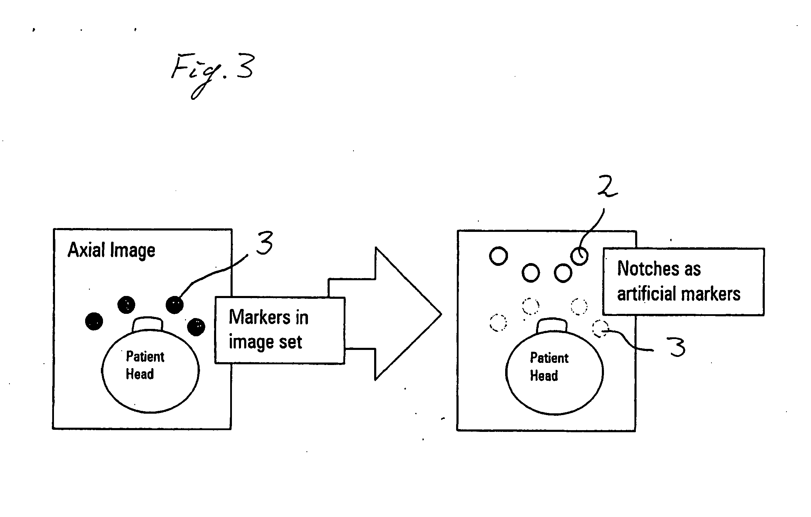

Fig. 3 shows a software module, which then can detect the registration structure markers 3 in the tomography and calculates a transformation matrix between the detected markers 3 in the image set and the well-known geometry of the registration structure 10. Thereafter the calculated transformation matrix is applied to the known position of the notches 2 and thereby they are transformed into the coordinate system of the image set. Artificial markers 2 are drawn at the exact position of the transformed notches in the image set. Then the registration structure markers 3 are deleted from the image set by simple blacking them out and the new image sets series for the IGS system is written in a standard image format (e.g. DICOM), which can be read by the IGS system.

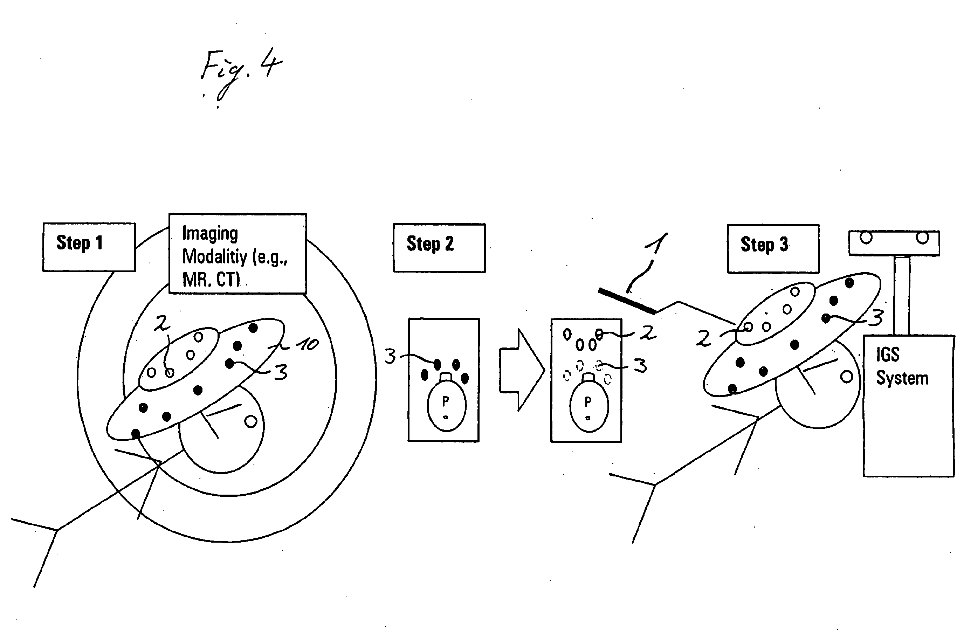

Fig. 4 shows a workflow in three steps. Step 1 displays the attachment of the registration structure 10 to the patient during the MRI and CT imaging process in such a way that at least half of the markers 3 of the registration structure 10 are imaged as well. In step 2 she image set is sent to the above described software module for processing. New image sets, containing the artificial markers 2, are then sent to the proprietary IGS system. In step 3 the proprietary IGS system detects artificially drawn markers and will ask user to point to the detected markers with a pointer 1 or any other trackable instrument, which also can have markers. The user simply identifies the artificial markers 2 by pointing with his tracked instrument 1 to the notches 2 of the registration structure 10, which will be 'easy-to-reach'.

As the MR/CT scanner is moved and the patient remains in his/her position, the operation is interrupted for some minutes only.

In this connection, it is also possible to create a postoperative data record for checking purposes. To do so, the MR/CT scanner is again moved into the operating theater while the patient is still anaesthetized and intubated. The data record, which will then be created anew, is pictorially represented and can be immediately checked by the surgery team. Such a final check-up is an important aid used to confirm that a complete tumor resection has taken place and to exclude acute complications, thereby considerably reducing the risk of having to perform a further operation in case the tumor should grow again. This also helps to reduce costs arising from repeated operations.

## Claims

1. Method for registering a body structure, wherein
- a reference structure comprising an imaging structure (8) having a plurality of markers (3) or connection systems (4) for markers (3) with markers (3) attached to the connection systems (4) for markers (3) and a plurality of artificial markers (2), wherein the artificial markers (2) are notches which are separately arranged from the connection systems (4) or the markers (3) and have a known relationship to the imaging structure (8), has been attached to the body;
- an imaging of the body or body structure is performed to obtain an image and the relationship between the body structure and the markers (3);
- the position of the artificial markers (2) is identified by the use of a trackable pointer (1);
- the markers (3) are deleted from the image; and
- the position of the body structure in space is identified using the identified position of the artificial markers (2) and the known relationship between the artificial markers (2) and the markers (3).

2. Method for registering a body structure according to the previous claim, wherein at least three markers (3) are imaged.

## Patentansprüche

1. Verfahren zum Registrieren einer Körperstruktur, wobei
- eine Referenzstruktur mit einer Abbildungsstruktur (8) mit einer Mehrzahl von Markern (3) oder Verbindungssystemen (4) für Marker (3) mit Markern (3), angebracht an den Verbindungssystemen (4) für Marker (3), und eine Mehrzahl von künstlichen Markern (2), wobei die künstlichen Marker (2) Einkerbungen sind, welche separat von den Verbindungssystemen (4) oder den Markern (3) angeordnet sind und ein bekanntes Lageverhältnis zu der Abbildungsstruktur (8) haben, an dem Körper befestigt wurde;
- eine Abbildung des Körpers oder der Körperstruktur durchgeführt wird, um eine Bild und das Lageverhältnis zwischen der Körperstruktur und den Markern (3) zu erhalten;
- die Position der künstlichen Marker (2) bestimmt wird durch die Verwendung eines trackbaren Pointers (1);
- die Marker (3) von dem Bild entfernt werden; und
- die Position der Köperstruktur im Raum bestimmt wird unter Verwendung der bestimmten Position der künstlichen Marker (2) und des bekannten Lageverhältnisses zwischen den künstlichen Markern (2) und den Markern (3).

2. Verfahren zum Registrieren einer Körperstruktur nach dem vorhergehenden Anspruch, wobei mindestens drei Marker (3) abgebildet werden.

## Revendications

1. Procédé de repérage d'une structure corporelle, dans lequel :
- une structure de référence comprenant une structure d'imagerie (8) comportant une pluralité de marqueurs (3) ou de systèmes de connexion (4) pour des marqueurs (3) avec des marqueurs (3) fixés aux systèmes de connexion (4) pour des marqueurs (3) et une pluralité de marqueurs artificiels (2), les marqueurs artificiels (2) étant des encoches qui sont disposées de façon séparée des systèmes de connexion (4) ou des marqueurs (3), et ayant une relation connue avec la structure d'imagerie (8), a été fixée au corps ;
- une imagerie du corps ou de la structure corporelle est effectuée afin d'obtenir une image et la relation entre la structure corporelle et les marqueurs (3) ;
- la position des marqueurs artificiels (2) est identifiée par l'utilisation d'un pointeur pouvant être suivi (1) ;
- les marqueurs (3) sont effacés de l'image ; et
- la position de la structure corporelle dans l'espace est identifiée à l'aide de la position identifiée des marqueurs artificiels (2) et de la relation connue entre les marqueurs artificiels (2) et les marqueurs (3).

2. Procédé de repérage d'une structure corporelle selon la revendication précédente, dans lequel on réalise une image d'au moins trois marqueurs (3).
